# EUROPEAN PATENT SPECIFICATION

(11) **EP 3 087 916 B1**
(45) Date of publication and mention of the grant of the patent: **20.09.2023**
(21) Application number: 15165300.3
(22) Date of filing: 28.04.2015
(51) Int. Cl.: A61B 5/024, A61B 5/021, A61B 5/08, A61B 5/1455

(54) **PHYSIOLOGICAL MEASUREMENT SENSOR**
SENSOR ZUR PHYSIOLOGISCHEN MESSUNG
CAPTEUR DE MESURE PHYSIOLOGIQUE

(43) Date of publication of application: 02.11.2016
(73) Proprietor: Nokia Technologies Oy, 02610 Espoo (FI)
(72) Inventor: Blomqvist, Kim, 02650 Espoo (FI); Jalkanen, Jukka, 01350 Vantaa (FI)
(74) Representative: Espatent Oy

(56) References cited:
- EP-A1- 1 444 948
- EP-A1- 1 815 786
- EP-B1- 1 051 106
- WO-A1-2015/176999
- JP-A- 2011 104 124
- US-A- 4 157 708
- US-A- 4 907 594
- US-A- 6 082 858
- US-A1- 2012 197 137
- US-A1- 2013 090 564

## Description

### TECHNICAL FIELD

The present application generally relates to physiological measurement sensors.

### BACKGROUND

This section illustrates useful background information without admission of any technique described herein representative of the state of the art.

Various metering devices that measure physiological conditions of users such as pulse sensors have become common for people to measure their own heart rate, movements or other parameters. The measurements can be performed using a chest strap that is worn under clothes or using a wrist worn watch-like sensor device.

Pulse or heart rate can be monitored for example optically using a photoplethymography (PPG) sensor. Optical heart rate measurement requires that the sensor is kept very stably on the wrist during the measurement as the photoplethysmographic (PPG) measurement is sensitive to all kind of movements of the sensor. Motion artifacts caused by sensor movements corrupt the pulsatile heart rate (HR) signal and confuse the HR monitoring algorithms of the sensor. The end result is that the calculated HR in beats per minute (bpm) is wrong.

However, keeping the sensor completely stably on the wrist is difficult in practice. For example, the wrist strap cannot be kept too tight, because it would be inconvenient / unpleasant for the user and might even stop or deteriorate blood circulation in small vessels thereby causing the measurement signal to disappear.

An optical heart rate sensor according to the state of the art is for instance described in US2012/0197137A1.

### SUMMARY

The invention is defined in the claims.

According to a first aspect of the present invention, there is provided a sensor according to claim 1.

In an example embodiment, the sensor further comprises a light source configured to emit light at the target wavelength.

In an example embodiment, the subtraction element is an analog subtraction circuit. In an example embodiment, the subtraction element is digital.

In an example embodiment, the sensor comprises a signal processing element configured to process the subtracted signal to produce the physiological measurement result. In an example embodiment, the first light detector and the second light detector comprise similar or identical characteristics. In an example embodiment, all characteristic of the light detectors are not necessarily similar.

In an example embodiment, the first light detector and the second light detector are integrated components on a common substrate.

In an example embodiment, the first light detector and the second light detector are connected in parallel with opposite polarities.

In an example embodiment, the sensor comprises light-scattering material arranged so that the light that enters the sensor passes through the light-scattering material prior to entering the first light detector and the second light detector.

In an example embodiment, the first light detector and the second light detector form a detector pair and the sensor comprises a plurality of said detector pairs forming a detector array.

In an example embodiment, the optical blocking filter is a notch filter or a band-stop filter.

In an example embodiment, the optical blocking filter is further enhanced with an additional band-pass filter.

According to a second aspect of the present invention, there is provided a user wearable apparatus according to claim 12.

According to a third aspect of the present invention, there is provided a method according to claim 13.

In an example embodiment, the method further comprises connecting the first light detector and the second light detector in parallel with opposite polarities.

Different non-binding example aspects have been illustrated in the foregoing. The embodiments in the foregoing are used merely to explain selected aspects or steps that may be utilized. Some embodiments may be presented only with reference to certain example aspects . It should be appreciated that corresponding embodiments may apply to other example aspects as well.

### BRIEF DESCRIPTION OF THE DRAWINGS

For a more complete understanding of example embodiments of the present invention, reference is now made to the following descriptions taken in connection with the accompanying drawings in which:
Fig. 1 is a simplified illustration of an example optical heart rate measurement;
Fig. 2 is a simplified illustration of an example physiological measurement sensor;
Fig. 3A is a schematic drawing illustrating an example embodiment;
Fig. 3B depicts example bandwidths of an example filter and light source;
Fig. 3C is a schematic drawing illustrating another example embodiment;
Figs. 4A and 4B are logical block diagrams of sensors of example embodiments;
Figs. 5-8 are circuit diagrams of example embodiments; and
Fig. 9 shows a flow chart of a process of an example embodiment.

### DETAILED DESCRIPTION OF THE DRAWINGS

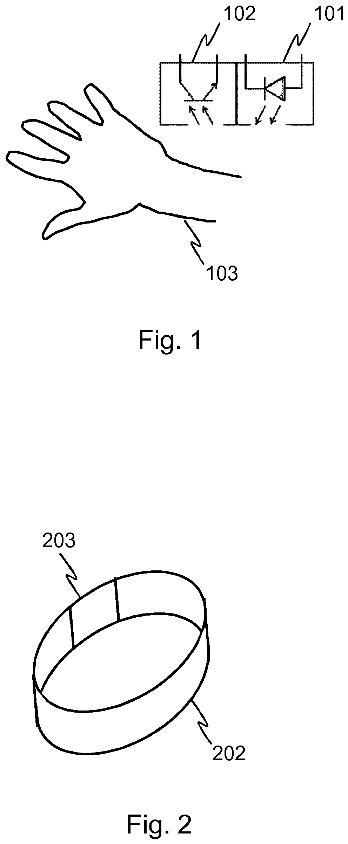

Example embodiments of the present invention and its potential advantages are understood by referring to Figs. 1 through 12 of the drawings. In this document, like reference signs denote like parts or steps.

In various example embodiments of the invention there is provided a new type of sensor for optical measurement of physiological conditions of a user. The sensor measures physiological conditions of a user and produces sensor signals corresponding to a property of the matter underlying the skin of the user (capillaries and veins, for example). The sensor is particularly suited for user wearable devices. In the following, various example embodiments are discussed in connection with optical heart rate sensors. Physiological conditions or physiological measurement results referred to herein may include one or more of the following: heart rate, respiration rate, blood pressure, oxygen saturation level, and glucose level. Also other physiological condition measurements may apply.

Heart rate can be monitored optically by measuring variations in blood volume with a photoplethymography (PPG) sensor. Fig. 1 is a simplified illustration of an example optical heart rate measurement. Fig. 1 shows a (reflective type) PPG sensor that comprises a LED (light emitting diode) 101, a light source, and a photo transistor 102, a light detector. Also a photo diode (PD) may be used as the light detector. The LED (optical emitter, light source) 101 emits light and the light detector 102 receives light rays reflected from a wrist 103 of a user. The sensor produces sensor signals based on the light detected by the light detector 102.

There is provided an optical sensor with two light detectors (e.g. photo diodes). In an embodiment one of the light detectors is sensitive to a range of wavelengths and the other one of the light detectors is insensitive to a certain target wavelength. The target wavelength is the wavelength one is interested in and the target wavelength may comprise a certain wavelength band.

One of the light detectors is covered with an optical filter that is configured to block some wavelengths or prevent some wavelengths from passing through the filter. The filter is a blocking filter. The filter may be for example a notch filter or a band-stop filter, such as a dichroic mirror/reflector. The filter is configured to block a target wavelength, which is the wavelength one is interested in. In an embodiment, the sensor comprises a light source that emits light at a certain wavelength. This wavelength is the target wavelength and the blocking filter matches the wavelength of the light source. That is, the blocking filter is configured to filter out or block the wavelength of the light source. In an example embodiment the light source is a green LED working at 525 nm peak wavelength and the blocking filter filters out the 525 nm wavelength. This is however only one example and other wavelengths can be equally used. The other one of the light detectors is used as is without additional optical band-stop filtering. That is, the other light detector detects a range of wavelengths.

Now, when the light detectors detect light, the detector that is insensitive to the target wavelengths and e.g. covered with the filter, detects less the target light than the other one. The detected light signals are subtracted from each other to produce a result signal that is cleared from noise and artifacts originating from unwanted wavelengths. In an embodiment there is an analog circuit configured to perform the subtraction. In another alternative the detected signals are analog-to-digital converted and then subtracted digitally. The resulting signal may then be used for producing a physiological measurement result, such as heart rate.

Fig. 2 is a simplified illustration of an example physiological condition sensor. The physiological condition sensor 203 is attached to a wrist strap 202 that allows the sensor 203 to be fitted around a wrist of the user.

The sensor 203 can be made of a suitable material, such as for example plastic (e.g. acrylonitrile butadiene styrene (ABS) or polycarbonate (PC)), carbon fiber materials, glass, wood, metal, ceramics or other material covered with fabric or leather or any combination of these. The strap may be made of suitable flexible or bendable material, such as plastic, fabric, and leather. In an example embodiment, the strap 202 and the sensor 203 are integrally formed of one piece of material. The material can comprise or consist of any of the following: plastics, metals, nano-fibers, carbon fiber, leather, fabric and glass.

Fig 2 shows the sensor attached to a wrist strap, but the sensor may equally be part of some other user wearable apparatus that can be fitted around a body part (e.g. wrist, ankle or finger) of a user. The sensor may be configured to be integrated into a garment of a user. The sensor may be attached or integrated for example to a belt, a sock, a shoe, a sleeve or a collar of a shirt or pullover, and/or a waistband of trousers or skirt. The sensor may be detachable from the garment. The sensor may be shaped like a watch and it may be configured to display time or other useful information to the user. The sensor may be attached to a patch/plaster (with adhesive) or to a ring. A further alternative is that the sensor is attached to an ear of the user. The sensor may be part of an earplug, for example.

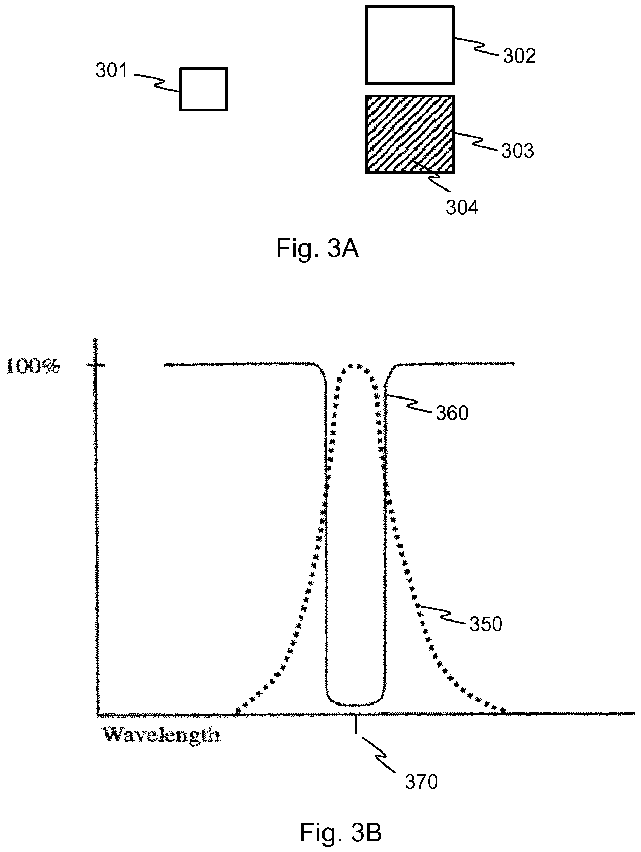

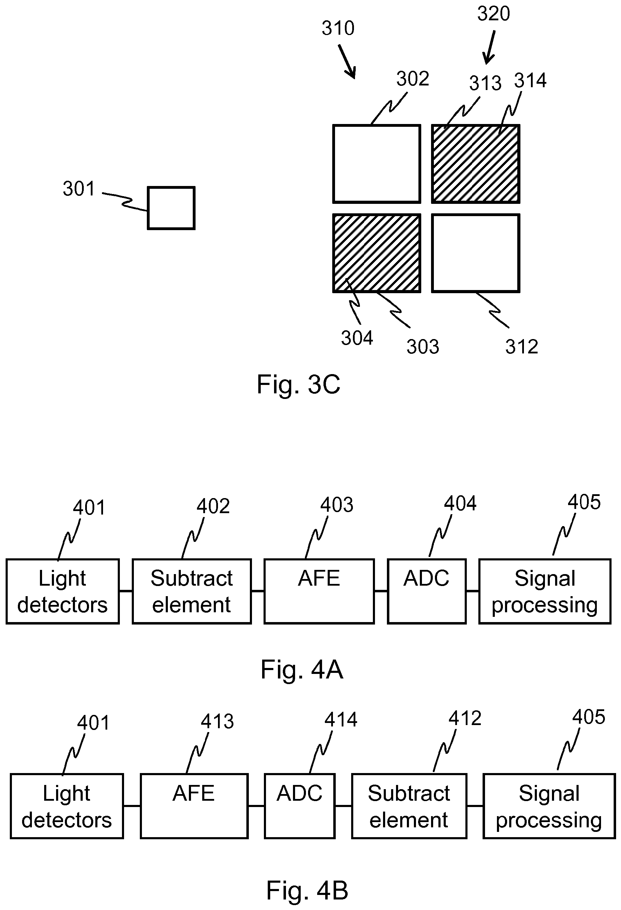

Fig. 3A is a schematic drawing illustrating an example. The aim is to detect signals at a certain target wavelength in a sensor. Figure 3 shows a light source 301 (e.g. a LED) and a first light detector 302 (e.g. a photo diode) and a second light detector 303 (e.g. a photo diode). The second light detector 303 is covered with a filter 304 that is configured to block or eliminate the target wavelength.

The filter 304 is for example a notch filter or a band-stop filter. In an embodiment the filter 304 is a dichroic mirror/reflector. Dichroic mirror/reflector can be very steep and therefore they may suit well embodiments of the invention.

The example of Fig 3A operates as follows: The light source 301 emits light. The light reflects from skin/tissue of a user and the reflected light arrives at the light detectors 302 and 303. The first light detector 302 detects all wavelengths of the reflected light. The second light detector 303 detects all other wavelengths of the reflected light except the wavelength(s) blocked by the filter 304. The first light detector 302 produces a first detected signal and the second light detector 303 produces a second detected signal. The second detected signal is then subtracted from the first detected signal. The subtraction can be done either in an analog circuit or digitally e.g. afterwards as a part of the digital signal processing. If analog subtraction is used, the subtraction may be performed at the very beginning of a chain of analog signal condition stage of sensor electronics e.g. before any amplification is applied. Whichever approach is chosen, the use of subtracted signal reduces the complexity of the needed digital correction, e.g. motion artifact compensation. In an embodiment the digital signal correction may not be necessarily needed at all.

In an embodiment the light detectors 302 and 303 are further covered with a band-pass filter that lets through mainly only the peak/target wavelength. In this way the detection results may be further improved as the band-pass filtering reduces the amount of unwanted wavelengths.

In an embodiment the light source emits light at a certain wavelength, e.g. green light with 525 nm peak wavelength, and the target is to detect this wavelength. In that case, the filter 304 is configured to block this certain wavelength, e.g. the wavelength 525 nm. Other wavelengths are possible, too, and even white light (broadband light source) or ambient light may be used.

Fig. 3B depicts example bandwidths of an example filter and light source. Dotted line 350 depicts bandwidth of a light source and solid line 360 depicts bandwidth of the filter. In the shown example the bandwidths of the light source and the filter happen to be similar. In alternative examples the bandwidth of the filter may be narrower and/or the bandwidth of the light source may be wider. A target wavelength 370, i.e. the wavelength of interest, comprises the center wavelength of the bandwidths. The target wavelength may comprise certain range near the center wavelength. The filter depicted in Fig. 3B is a band-stop filter. A notch filter would be sharper and the bandwidth of a notch filter would be narrower than the depicted bandwidth.

In an example embodiment the sensor does not include the light source 301. Instead ambient light is used for sensing the physiological conditions in the sensor. For example ambient light reflected from the skin/tissue of the user is detected by the light detectors 302 and 303.

Fig. 3C is a schematic drawing illustrating another example embodiment comprising a plurality of detector pairs. Figure 3C shows a light source 301, a first light detector 302, a second light detector 303, a third light detector 312, and a fourth light detector 313. The light source is for example a LED and the light detectors are for example photo diodes. The second light detector 303 is covered with a filter 304 that is configured to block or eliminate a certain wavelength. The first light detector 302 and the second light detector 303 from a first detector pair 310. The fourth light detector 313 is covered with a filter 314 that is configured to block or eliminate a certain wavelength. The third light detector 312 and the fourth light detector 313 from a second detector pair 320.

Detection of signals by each detector pair in Fig. 3C is performed in a similar manner as disclosed in the foregoing in connection with Fig. 3A.

The aim is to detect signals at a certain target wavelength and the filters 304 and 314 are configured to block this target wavelength. Resulting signals obtained from different detector pairs 310, 320 may be combined in a suitable manner. For example, average of the resulting signals from different detector pairs can be used.

In an embodiment different detector pairs 310, 320 are configured to detect different wavelengths, that is, there may be more than one target wavelength to be detected. In this case there is at least one detector pair for each target wavelength and the filter comprised in different detector pairs matches the target wavelength of that particular detector pair. In this case the sensor may comprise multiple light sources, each of which emits different wavelength or there may be only one light source. In prior art solutions, different wavelengths are typically detected sequentially. That is, it has been feasible to detect only one wavelength at a time. With the solution of various embodiments of the invention, it is possible to detect different wavelengths at the same time as the disclosed structure efficiently removes unwanted wavelengths from the final detected signal. Therefore the detector pairs 310, 320 are sufficiently wavelength selective to allow simultaneous detection of different wavelengths.

Fig. 3C shows two detector pairs, but there may be even more detector pairs. The detector pairs may form an array of detectors.

In an embodiment the light detectors in Figs. 3A and 3C may be covered with a light-scattering material to evenly spread out the incoming light to all light detectors. The light-scattering material may be a light-scattering material film or lens or optical diffuser.

Figs. 4A and 4B are logical block diagrams of sensors. A chain of logical processing blocks is illustrated. It is to be noted that the shown logical blocks do not necessarily correspond to separate physical blocks. Instead functionality of a plurality of logical blocks may be implemented in one physical electronic circuit, for example. Furthermore, the order of the logical blocks may vary from the shown examples. Fig. 4A illustrates an analog subtraction example and Fig. 4B illustrates a digital subtraction example.

The sensor of Fig. 4A comprises a light detector block 401, a subtraction element 402, an analog front-end (AFE) block 403, an analog-to-digital conversion (ADC) block 404 and a signal processing block 405 connected into a chain. The light detector block 401 comprises two light detectors (or even more than two light detectors as disclosed in connection with Fig. 3C). The subtraction element 402 is an analog circuit that combines the signals detected by the light detectors and produces a subtracted signal. The analog front-end block 403 performs signal processing in analog domain. For example amplification, filtering and/or conditioning may be performed. The signal is analog-to-digital converted in block 404. The signal processing block 405 produces final physiological measurement results (e.g. heart rate) based on the sensor signal obtained from blocks 401-404. The signal processing block 405 may apply further correction algorithms to the sensor signal if necessary.

The sensor of Fig. 4B comprises a light detector block 401, an analog front-end (AFE) block 413, an analog-to-digital conversion block 414, a subtraction element 412 and a signal processing block 405 connected into a chain. The light detector block 401 comprises two light detectors (or even more than two light detectors as disclosed in connection with Fig. 3C). The signals detected by the two light detectors in the light detector block 401 are separately processed in the analog front-end block 414 (e.g. amplified, filtered and/or conditioned) and analog-to-digital converted in block414 and the two signals are combined in the digital subtraction element 412. The subtraction element 412 is digital in this example. The subtraction element may be part of the signal processing block 405. The signal processing block 405 produces final physiological measurement results (e.g. heart rate) based on the sensor signal obtained from blocks 401, 413, 414 and 412. The signal processing block 405 may apply further correction algorithms to the sensor signal if necessary.

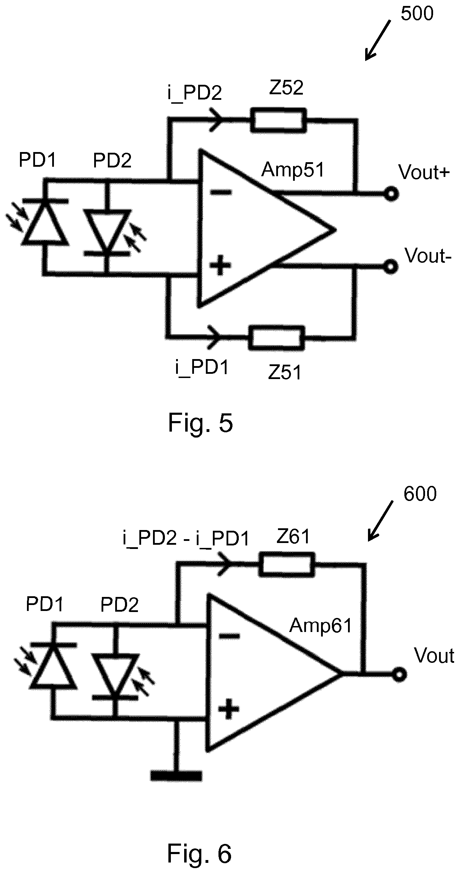

Figs. 5-8 are circuit diagrams of example embodiments. In the shown examples there are two photo diodes PD1 and PD2 operating as the light detectors.

In Figs. 5-6 the photo diodes PD1 and PD2 are arranged in parallel connection with opposite polarities. That is, the photo diodes are connected back-to-back in parallel. It is feasible to use such back-to-back arrangement in the shown example circuits as the feedback mechanism of the amplifier maintains zero bias (DC) across both photo diodes PD1 and PD2. In this way PD1 does not get reverse biased and PD2 does not get forward biased, or vice versa.

Fig. 5 shows a fully differential detection circuit 500. The circuit comprises the photo diodes PD1 and PD2, two impedances Z51 and Z52 and an amplifier Amp51. As an output the circuit 500 provides voltages Vout- and Vout+ representative of the difference between light detected by the photo diodes PD1 and PD2. In an example, the circuit of Fig. 5 implements the blocks 401, 402 and part of the 403 of Fig. 4A.

Fig. 6 shows a single-ended detection circuit 600. The circuit comprises the photo diodes PD1 and PD2, an impedance Z61 and an amplifier Amp61. As an output the circuit 600 provides voltage Vout representative of difference between the light detected by the photo diodes PD1 and PD2. In an example, the circuit of Fig. 6 implements the blocks 401, 402 and part of the 403 of Fig. 4A.

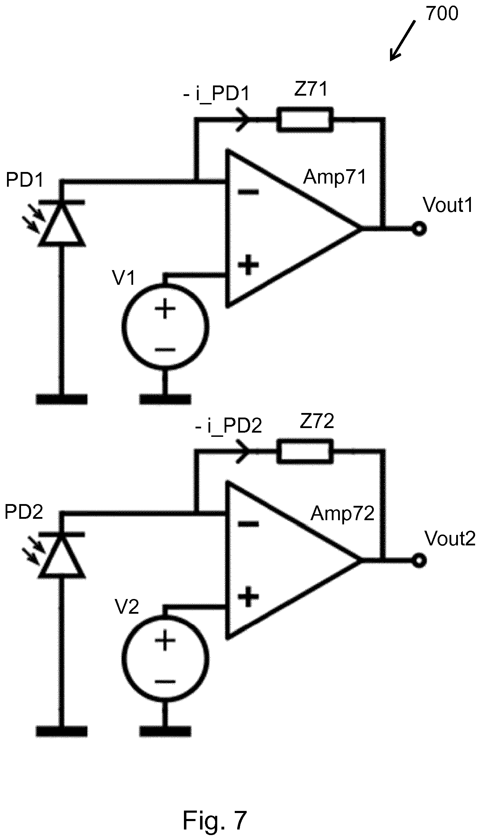

Fig. 7 shows a single-ended detection circuit 700 with optional biasing and separated outputs. Photoconductive mode is shown in Fig. 7. The circuit comprises the photo diodes PD1 and PD2, voltage sources V1 and V2, impedances Z71 and Z72, and amplifiers Amp71 and Amp72. As an output the circuit 700 provides voltages Vout1 and Vout2 representative of the light detected by the photo diodes PD1 and PD2 respectively. In an example, the circuit of Fig. 7 implements the block 401 of Fig. 4A.

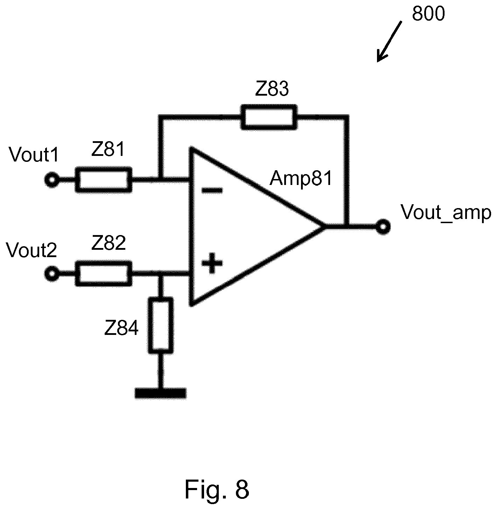

Fig. 8 shows a difference amplifier 800. The circuit comprises impedances Z81-Z84 and an amplifier Amp81. The outputs Vout1 and Vout2 of the circuit of Fig. 7 may be connected to inputs of the difference amplifier 800. The difference amplifier 800 produces an output voltage Vout_amp that is representative of the difference between the voltages Vout1 and Vout2. In an example, the circuit of Fig. 8 implements the blocks 402 and part of the 403 of Fig. 4A. That is, the circuits of Figs. 7 and 8 implement the blocks 401-403 of Fig. 4A.

It is to be noted that the Figs. 5-8 do not show the optical blocking filter in front of or on top of one of the photo diodes PD1 and PD2. Clearly such filter is included in practical implementations.

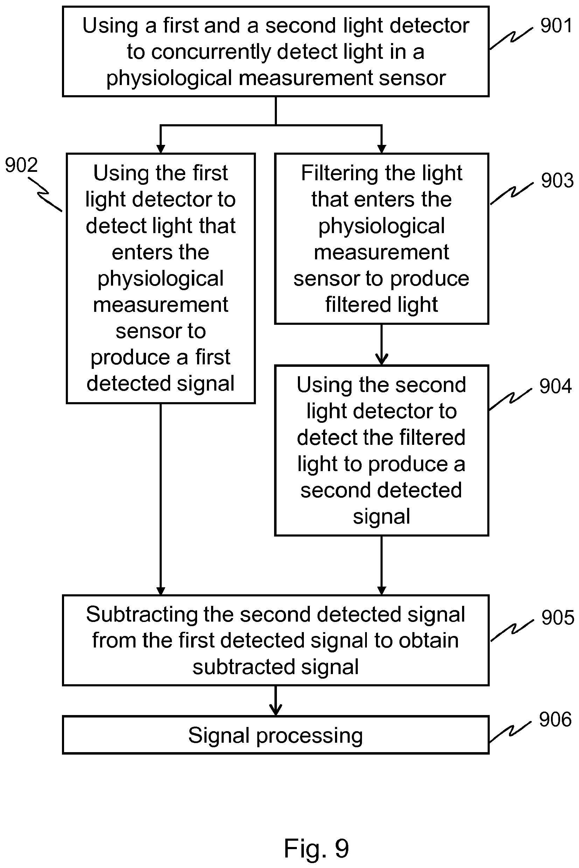

Fig. 9 shows a flow chart of a process.

The process comprises:
901: A first and a second light detector are used to concurrently detect light in a an optical heart rate sensor. In an embodiment the first and second light detectors are photo diodes arranged into a parallel connection with opposite polarities (a back-to-back arrangement). The diodes may be discrete diodes arranged e.g. in the back-to-back arrangement on a PWB or the diodes may be manufactured as a package of diode pairs readily arranged in the back-to-back arrangement. In an embodiment, the photo diodes are substantially identical or have substantially matching electrical characteristics or at least comprise similar characteristics. The photo diodes may be manufactured for example next to each other on the same (silicon) wafer/substrate to ensure similar characteristics. Other alternatives are manufacturing similar photo diodes on the same die, manufacturing similar photo diodes using separate dies (the separate dies having similar characteristics) or using binning manufactured components. It is noted that one may equally produce more than two photo diodes that comprise identical/similar characteristics.
902: The first light detector detects light that enters the physiological measurement sensor and produces a first detected signal.
903: The light that enters the physiological condition sensor is filtered to produce filtered light. The filtering is performed e.g. using a notch/band-stop filter, such as a dichroic mirror/reflector.
904: The second light detector detects the filtered light and produces a second detected signal.
905: The second detected signal is subtracted from the first detected signal to obtain subtracted signal. The subtraction is performed for example using analog components or done afterwards in digital domain.
906: The subtracted signal is then further processed to produce final physiological measurements results.

Without in any way limiting the scope, interpretation, or application of the claims appearing below, a technical effect of one or more of the example embodiments disclosed herein is that an improved optical sensor is provided. For example analog solution provided in various example embodiments may be faster and more robust than digital solutions. Another technical effect of one or more of the example embodiments disclosed herein is that a need to develop and use complex software algorithms to correct the measurement signal may be reduced. Instead simpler algorithms may be applied.

Another technical effect of one or more of the example embodiments disclosed herein is that DC component of a measured signal is reduced. In heart rate monitoring applications DC is not an interesting component and the presence of the DC component only narrows down the effective dynamic range of an analog front-end.

Another technical effect of one or more of the example embodiments disclosed herein is that the solution is easy to take into use. Various example solutions are compatible with existing optical measurement ICs (integrated circuits) and/or can be easily made compatible with existing optical measurement ICs.

Another technical effect of one or more of the example embodiments disclosed herein is that concurrent measurement using two or more wavelengths is enabled. The photo diode configuration of various embodiments makes the photo diode extremely wavelength selective and therefore it is possible to measure two or more wavelengths at the same time. In other solutions time multiplexing is often utilized for multi-wavelength measurements, that is, different wavelengths are measured at different time periods.

Another technical effect of one or more of the example embodiments disclosed herein is that the solution is less sensitive to ambient and other unwanted light sources. Another technical effect of one or more of the example embodiments disclosed herein is that a wide spectrum light source can be used. For example wider spectrum than spectrum provided by LEDs could be used.

If desired, the different functions discussed herein may be performed in a different order and/or concurrently with each other. Furthermore, if desired, one or more of the before-described functions may be optional or may be combined.

Although various aspects of the invention are set out in the independent claims, other aspects of the invention comprise combinations of features from the the dependent claims with the features of the independent claims.

It is also noted herein that while the foregoing describes examples, these descriptions should not be viewed in a limiting sense. Rather, there are several variations and modifications which may be made without departing from the scope of the present invention as defined in the appended claims.

## Claims

1. An optical heart rate sensor configured to detect a certain target wavelength, the sensor comprising:
a first light detector (302, 312) and a second light detector (303, 313) configured to concurrently detect light reflected from skin/tissue of a user, and
an optical blocking filter (304, 314) configured to block the target wavelength, wherein
the first light detector (302, 312) is configured to detect all wavelengths of the light that enters the sensor to produce a first detected signal,
the optical blocking filter (304, 314) is configured to filter the light that enters the sensor to produce filtered light, and
the second light detector (303, 313) is configured to detect the filtered light to detect all other wavelengths of the light that enters the sensor except the target wavelength to produce a second detected signal, the sensor further comprising
a subtraction element (402, 412) configured to subtract the second detected signal from the first detected signal to produce a subtracted signal, wherein the subtracted signal is a sensor signal usable for producing the heart rate as physiological measurement result.

2. The sensor of claim 1, further comprising
a light source (301) configured to emit light at the target wavelength.

3. The sensor of any preceding claim, wherein the subtraction element (402) is an analog subtraction circuit.

4. The sensor of any one of claims 1-2, wherein the subtraction element (412) is digital.

5. The sensor of any preceding claim, wherein the sensor comprises a signal processing element (405) configured to process the subtracted signal to produce the physiological measurement result.

6. The sensor of any preceding claim, wherein the first light detector (302, 312) and the second light detector (303, 313) comprise similar or identical characteristics.

7. The sensor of any preceding claim, wherein the first light detector (302, 312) and the second light detector (303, 313) are integrated components on a common substrate.

8. The sensor of any preceding claim, wherein the first light detector (302, 312) and the second light detector (303, 313) are connected in parallel with opposite polarities.

9. The sensor of any preceding claim, wherein the sensor comprises light-scattering material arranged so that the light that enters the sensor passes through the light-scattering material prior to entering the first light detector and the second light detector.

10. The sensor of any preceding claim, wherein the first light detector (302, 312) and the second light detector (303, 313) form a detector pair and the sensor comprises a plurality of said detector pairs forming a detector array.

11. The sensor of any preceding claim, wherein the optical blocking filter (304, 314) is a notch filter or a band-stop filter.

12. A user wearable apparatus (202) comprising the sensor of any preceding claim.

13. A method in an optical heart rate sensor configured to detect a certain target wavelength, the method comprising:
using (901) a first and a second light detector to concurrently detect light reflected from skin/tissue of a user in a physiological measurement sensor,
using (902) the first light detector to detect all wavelengths of light that enters the physiological measurement sensor to produce a first detected signal,
filtering (903) the light that enters the physiological measurement sensor by blocking the target wavelength with an optical blocking filter to produce filtered light,
using (904) the second light detector to detect the filtered light to detect all other wavelengths of the light that enters the sensor except the target wavelength to produce a second detected signal,
subtracting (905) the second detected signal from the first detected signal to obtain a subtracted signal, wherein the subtracted signal is a sensor signal usable for producing the heart rate as physiological measurement result.

14. The method of claim 13, further comprising:
connecting the first light detector and the second light detector in parallel with opposite polarities.

## Patentansprüche

1. Optischer Herzfrequenzsensor, der dazu ausgelegt ist, eine bestimmte Zielwellenlänge zu detektieren, wobei der Sensor Folgendes umfasst:
einen ersten Lichtdetektor (302, 312) und einen zweiten Lichtdetektor (303, 313), die dazu ausgelegt sind, Licht, das von einer Haut bzw. einem Gewebe eines Benutzers reflektiert wird, zusammen zu detektieren, und
ein optisches Sperrfilter (304, 314), das dazu ausgelegt ist, die Zielwellenlänge zu sperren, wobei
der erste Lichtdetektor (302, 312) dazu ausgelegt ist, alle Wellenlängen des Lichts, das in den Sensor eintritt, zu detektieren, um ein erstes detektiertes Signal zu produzieren,
das optische Sperrfilter (304, 314) dazu ausgelegt ist, das Licht, das in den Sensor eintritt, zu filtern, um gefiltertes Licht zu produzieren, und
der zweite Lichtdetektor (303, 313) dazu ausgelegt ist, das gefilterte Licht zu detektieren, um alle anderen Wellenlängen des Lichts, das in den Sensor eintritt, außer der Zielwellenlänge zu detektieren, um ein zweites detektiertes Signal zu produzieren, wobei der Sensor ferner Folgendes umfasst
ein Subtraktionselement (402, 412), das dazu ausgelegt ist, das zweite detektierte Signal vom ersten detektierten Signal zu subtrahieren, um ein subtrahiertes Signal zu produzieren, wobei das subtrahierte Signal ein Sensorsignal ist, das zum Produzieren der Herzfrequenz als ein physiologisches Messergebnis verwendbar ist.

2. Sensor nach Anspruch 1, der ferner Folgendes umfasst
eine Lichtquelle (301), die dazu ausgelegt ist, Licht mit der Zielwellenlänge zu emittieren.

3. Sensor nach einem der vorhergehenden Ansprüche, wobei das Subtraktionselement (402) eine analoge Subtraktionsschaltung ist.

4. Sensor nach einem der Ansprüche 1-2, wobei das Subtraktionselement (412) digital ist.

5. Sensor nach einem der vorhergehenden Ansprüche, wobei der Sensor ein Signalverarbeitungselement (405) umfasst, das dazu ausgelegt ist, das subtrahierte Signal zu verarbeiten, um das physiologische Messergebnis zu produzieren.

6. Sensor nach einem der vorhergehenden Ansprüche, wobei der erste Lichtdetektor (302, 312) und der zweite Lichtdetektor (303, 313) ähnliche oder identische Eigenschaften umfassen.

7. Sensor nach einem der vorhergehenden Ansprüche, wobei der erste Lichtdetektor (302, 312) und der zweite Lichtdetektor (303, 313) integrierte Komponenten auf einem gemeinsamen Substrat sind.

8. Sensor nach einem der vorhergehenden Ansprüche, wobei der erste Lichtdetektor (302, 312) und der zweite Lichtdetektor (303, 313) parallel mit entgegengesetzten Polaritäten verbunden sind.

9. Sensor nach einem der vorhergehenden Ansprüche, wobei der Sensor ein Lichtstreuungsmaterial umfasst, das derart angeordnet ist, dass das Licht, das in den Sensor eintritt, durch das Lichtstreuungsmaterial geleitet wird, bevor es in den ersten Lichtdetektor und den zweiten Lichtdetektor eintritt.

10. Sensor nach einem der vorhergehenden Ansprüche, wobei der erste Lichtdetektor (302, 312) und der zweite Lichtdetektor (303, 313) ein Detektorpaar bilden und der Sensor eine Vielzahl der Detektorpaare umfasst, die ein Detektorarray bilden.

11. Sensor nach einem der vorhergehenden Ansprüche, wobei das optische Sperrfilter (304, 314) ein Kerbfilter oder ein Bandstoppfilter ist.

12. Von einem Benutzer tragbare Vorrichtung (202), die den Sensor nach einem der vorhergehenden Ansprüche umfasst.

13. Verfahren in einem optischen Herzfrequenzsensor, der dazu ausgelegt ist, eine bestimmte Zielwellenlänge zu detektieren, wobei das Verfahren Folgendes umfasst:
Verwenden (901) eines ersten und eines zweiten Lichtdetektors, um zusammen Licht zu detektieren, das von einer Haut bzw. einem Gewebe eines Benutzers in einem physiologischen Messsensor reflektiert wird,
Verwenden (902) des ersten Lichtdetektors, um alle Wellenlängen von Licht zu detektieren, das in den physiologischen Messsensor eintritt, um ein erstes detektiertes Signal zu produzieren,
Filtern (903) des Lichts, das in den physiologischen Messsensor eintritt, durch Sperren der Zielwellenlänge mit einem optischen Sperrfilter, um gefiltertes Licht zu produzieren,
Verwenden (904) des zweiten Lichtdetektors, um das gefilterte Licht zu detektieren, um alle anderen Wellenlängen des Lichts, das in den Sensor eintritt, außer der Zielwellenlänge zu detektieren, um ein zweites detektiertes Signal zu produzieren,
Subtrahieren (905) des zweiten detektierten Signals vom ersten detektierten Signal, um ein subtrahiertes Signal zu erhalten, wobei das subtrahierte Signal ein Sensorsignal ist, das zum Produzieren der Herzfrequenz als ein physiologisches Messergebnis verwendbar ist.

14. Verfahren nach Anspruch 13, das ferner Folgendes umfasst:
paralleles Verbinden des ersten Lichtdetektors und des zweiten Lichtdetektors mit entgegengesetzten Polaritäten.

## Revendications

1. Capteur de fréquence cardiaque optique configuré pour détecter une certaine longueur d'onde cible, le capteur comprenant :
un premier détecteur de lumière (302, 312) et un second détecteur de lumière (303, 313) configurés pour détecter simultanément une lumière réfléchie depuis une peau/un tissu d'un utilisateur, et
un filtre bloquant optique (304, 314) configuré pour bloquer la longueur d'onde cible, dans lequel
le premier détecteur de lumière (302, 312) est configuré pour détecter toutes les longueurs d'onde de la lumière qui entre dans le capteur afin de produire un premier signal détecté,
le filtre bloquant optique (304, 314) est configuré pour filtrer la lumière qui entre dans le capteur afin de produire une lumière filtrée, et
le second détecteur de lumière (303, 313) est configuré pour détecter la lumière filtrée afin de détecter toutes les autres longueurs d'onde de la lumière qui entre dans le capteur à l'exception de la longueur d'onde cible en vue de produire un second signal détecté, le capteur comprenant en outre
un élément de soustraction (402, 412) configuré pour soustraire le second signal détecté du premier signal détecté afin de produire un signal soustrait, dans lequel le signal soustrait est un signal de capteur utilisable pour produire la fréquence cardiaque en tant que résultat de mesure physiologique.

2. Capteur selon la revendication 1, comprenant en outre une source de lumière (301) configurée pour émettre une lumière à la longueur d'onde cible.

3. Capteur selon l'une quelconque des revendications précédentes, dans lequel l'élément de soustraction (402) est un circuit de soustraction analogique.

4. Capteur selon l'une quelconque des revendications 1-2, dans lequel l'élément de soustraction (412) est numérique.

5. Capteur selon l'une quelconque des revendications précédentes, dans lequel le capteur comprend un élément de traitement de signal (405) configuré pour traiter le signal soustrait afin de produire le résultat de mesure physiologique.

6. Capteur selon l'une quelconque des revendications précédentes, dans lequel le premier détecteur de lumière (302, 312) et le second détecteur de lumière (303, 313) comprennent des caractéristiques similaires ou identiques.

7. Capteur selon l'une quelconque des revendications précédentes, dans lequel le premier détecteur de lumière (302, 312) et le second détecteur de lumière (303, 313) sont des composants intégrés sur un substrat commun.

8. Capteur selon l'une quelconque des revendications précédentes, dans lequel le premier détecteur de lumière (302, 312) et le second détecteur de lumière (303, 313) sont reliés en parallèle avec des polarités opposées.

9. Capteur selon l'une quelconque des revendications précédentes, dans lequel le capteur comprend un matériau diffusant la lumière agencé de sorte que la lumière qui entre dans le capteur passe à travers le matériau diffusant la lumière avant d'entrer dans le premier détecteur de lumière et le second détecteur de lumière.

10. Capteur selon l'une quelconque des revendications précédentes, dans lequel le premier détecteur de lumière (302, 312) et le second détecteur de lumière (303, 313) constituent une paire de détecteurs et le capteur comprend une pluralité desdites paires de détecteurs constituant un réseau de détecteurs.

11. Capteur selon l'une quelconque des revendications précédentes, dans lequel le filtre bloquant optique (304, 314) est un filtre d'absorption ou un filtre coupe-bande.

12. Appareil pouvant être porté par un utilisateur (202) comprenant le capteur selon l'une quelconque des revendications précédentes.

13. Procédé dans un capteur de fréquence cardiaque optique configuré pour détecter une certaine longueur d'onde cible, le procédé comprenant :
l'utilisation (901) d'un premier et d'un second détecteur de lumière pour détecter simultanément une lumière réfléchie depuis une peau/un tissu d'un utilisateur dans un capteur de mesure physiologique,
l'utilisation (902) du premier détecteur de lumière pour détecter toutes les longueurs d'onde de lumière qui entre dans le capteur de mesure physiologique afin de produire un premier signal détecté,
le filtrage (903) de la lumière qui entre dans le capteur de mesure physiologique en bloquant la longueur d'onde cible avec un filtre bloquant optique pour produire une lumière filtrée,
l'utilisation (904) du second détecteur de lumière pour détecter la lumière filtrée afin de détecter toutes les autres longueurs d'onde de la lumière qui entre dans le capteur à l'exception de la longueur d'onde cible en vue de produire un second signal détecté,
la soustraction (905) du second signal détecté du premier signal détecté pour obtenir un signal soustrait, dans lequel le signal soustrait est un signal de capteur utilisable pour produire la fréquence cardiaque en tant que résultat de mesure physiologique.

14. Procédé selon la revendication 13, comprenant en outre :
la liaison du premier détecteur de lumière et du second détecteur de lumière en parallèle avec des polarités opposées.
